(19) **Europäisches Patentamt**

**European Patent Office**

**Office européen des brevets**

(11) **EP 0 874 616 B1**

(12) **EUROPÄISCHE PATENTSCHRIFT**

(45) Veröffentlichungstag und Bekanntmachung des Hinweises auf die Patenterteilung:
**28.11.2001 Patentblatt 2001/48**

(51) Int Cl.[7]: **A61K 7/16**, A61K 7/22, A61K 7/18

(21) Anmeldenummer: **97900568.3**

(22) Anmeldetag: **07.01.1997**

(86) Internationale Anmeldenummer:
**PCT/EP97/00033**

(87) Internationale Veröffentlichungsnummer:
**WO 97/25966 (24.07.1997 Gazette 1997/32)**

(54) **ZAHNPFLEGEMITTEL**

DENTAL HYGIENE AGENTS

PRODUITS D'HYGIENE DENTAIRE

(84) Benannte Vertragsstaaten:
**AT BE CH DE DK ES FI FR GB GR IT LI NL PT SE**

(30) Priorität: **15.01.1996 DE 19601155**

(43) Veröffentlichungstag der Anmeldung:
**04.11.1998 Patentblatt 1998/45**

(73) Patentinhaber: **Henkel Kommanditgesellschaft auf Aktien**
**40589 Düsseldorf-Holthausen (DE)**

(72) Erfinder:
• **WÜLKNITZ, Peter**
**D-42799 Leichlingen (DE)**
• **ARENDS, Joop**
**NL-9765 AP Paterswolde (NL)**

(56) Entgegenhaltungen:
**EP-A- 0 414 128          WO-A-91/15189**
**DE-U- 9 409 162**

EP 0 874 616 B1

EP 0 874 616 B1

**Beschreibung**

Die Erfindung betrifft Mund- und Zahnpflegemittel mit einem Gehalt an kationischen antimikrobiellen Wirkstoffen, die aufgrund spezifischer Komponenten in der Formulierung verstärkt auf der Zahnoberfläche und in den Zahnbelägen angereichert werden.

Durch die üblichen Maßnahmen der Mundhygiene werden Zahnbeläge - insbesondere an schwer zugänglichen Stellen zwischen den Zähnen, am Zahnfleischsaum und in Zahnfissuren nicht ausreichend entfernt. In diesen Belägen können Bakterien weiter aktiv sein und Zahnfleischentzündungen oder Karies verursachen. Darüber hinaus wirken diese Beläge als Biofilme, die eine Diffüsionsbarriere für viele Wirkstoffe darstellen, die bei der Mund- und Zahnpflege eingesetzt werden. Dadurch wird die Wirkung von z.B. Fluoriden oder antimikrobiellen Wirkstoffen erheblich gemindert.

Aus der europäischen Patentschrift EP-A-185 971 war schon bekannt, daß die Wirksamkeit antibakterieller Biguanidverbindungen durch eine Kombination mit Alkylglycosid-Tensiden gesteigert werden kann. Es war jedoch bisher nicht bekannt, daß die Adsorption der kationischen antimikrobiellen Biguanidverbindungen an die kariogenen Biofilme noch erheblich weiter erhöht werden kann.

Gegenstand der Erfindung sind Mund- und Zahnpflegemittel mit einem Gehalt an wasserlöslichen kationischen Wirkstoffen, die zur Anreicherung dieser Wirkstoffe in den kariogenen Biofilmen der Mund- und Zahnoberfläche zusätzlich ein Alkyl-(oligo)-glucosid der Formel $RO(C_6H_{10}O_5)_x$-H, in der R eine Alkylgruppe mit 8 bis 16 C-Atomen, $(C_6H_{10}O_5)$ ein Glucosidrest und x dessen Oligomerationsgrad von 1 bis 10 ist, sowie ein wasserlösliches Monofluorophosphat-Salz enthalten.

Die adsorptionserhöhende Wirkung geht dabei nur unwesentlich von den Monofluorophosphatsalzen allein aus, sie wird vielmehr durch die gemeinsame Wirkung der Alkylglycosid-Tenside und der Monofluorophosphatsalze um ein mehrfaches gesteigert.

Als wasserlösliche kationische, antimikrobiell wirksame Stoffe eignen sich z.B. oberflächenaktive Amine und quartäre Ammoniumverbindungen wie z.B. Cetyltrimethylammonium-bromid, Benzethoniumchlorid, Cetylpyridiniumchlorid oder das als Aminfluorid 297 bekannte N,N,N'-tris-(2-Hydroxyethyl)-N'-octadecyl-1,3-diaminopropan-diohydrofluorid.

Bevorzugt sind jedoch die antimikrobiell wirksamen Biguanidverbindungen wie z.B. das Polyhexamethylenbiguanid des Typs Vantocil® IB (ICI) oder die aus US-A-2 684 924, US-A-2 990 425, US-A-3,468 898, US-A-4 022 834, US-A-4 053 636 und US-A-4 198 392 bekannten antimikrobiellen Biguanid-Verbindungen geeignet.

Besonders bevorzugt eignet sich das aus GB-A-705 838 bekannte 1,1'-Hexamethylen-bis-(4-chlorphenyl)-biguanid ("Chlorhexidin") in Form eines wasserlöslichen, verträglichen Salzes, z.B. in Form des Acetats oder Gluconats.

Alkyl-(oligo)-glycoside, ihre Herstellung und Verwendung als oberflächenaktive Stoffe sind beispielsweise aus US-A-3 839 318, US-A-3 707 535, US-A-3 547 828 DE-A-19 43 689, DE-A-20 36 472 und DE-A-30 01 064 sowie EP-A-77 167 bekannt. Bezüglich des Glycosidrestes gilt, daß sowohl Monoglycoside (x = 1), bei denen ein Hexoserest glycosidisch an einen Fettalkohol mit 10 bis 16 C-Atomen gebunden ist, als auch oligomere Glycoside mit einem Oligomerisationsgrad x bis 10 geeignet sind. Der Oligomerisationsgrad ist dabei ein statistischer Mittelwert, dem eine für solche technischen Produkte übliche Homologenverteilung zugrunde liegt.

Bevorzugt eignet sich als Alkyl-(oligo)-glycosid ein Alkyl-(oligo)-glucosid der Formel $RO(C_6H_{10}O_5)_x$-H, in der R eine Alkylgruppe mit 12 bis 14 C-Atomen ist und x einen Mittelwert von 1 bis 4 hat.

Als wasserlösliche Monofluorophosphat-Salze eignen sich z.B. die Alkali- oder Ammoniumsalze. Bevorzugt werden das Natrium-monofluorophosphat ($Na_2PO_3F$) und das Kaliummonofluorophosphat ($K_2PO_3F$) eingesetzt.

Die erfindungsgemäße verstärkte Adsorption der kationischen Wirkstoffe an die Zahnpellicle ermöglicht eine sehr sparsame Verwendung dieser Stoffe. Einsatzmengen im Bereich von 0,004 bis 0,5 Gew.-% der kationischen antimikrobiellen Wirkstoffe, bezogen auf die Zusammensetzung des Zahnpflegemittels, sind daher ausreichend.

Auch das Alkylglycosid-Tensid kann sehr sparsam verwendet werden. Zur Erreichung der erfindungsgemäßen Steigerung der Adsorption der kationischen antimikrobiellen Wirkstoffe am Zahnbelag sind bereits Mengen von 0,005 bis 1 Gew.-% eines Alkyl-(oligo)-glucosids ausreichend.

Auch das wasserlösliche Fluorophosphat-Salz wirkt schon in relativ niedriger Konzentration, z.B. ist das Na-Monofluorophosphat-Salz schon in einer Menge von 0,003 bis 1,2 Gew.-% in der Zusammensetzung ausreichend wirksam.

Daraus ergibt sich für ein bevorzugtes Mund- und Zahnpflegemittel gemäß der vorliegenden Erfindung ein Gehalt von

(A) 0.004 bis 0,5 Gew.-% eines kationischen antimikrobiellen Wirkstoffs
(B) 0,005 bis 1 Gew.-% eines Alkyl-(oligo)-glycosids und
(C) 0,003 bis 1,2 Gew.-% eines Monofluorophosphat-Salzes (berechnet als Na-Salz).

Die erfindungsgemäßen Mund- und Zahnpflegemittel können als Mundwasser. Gel, flüssige Zahnputzlotion oder steife Zahnpaste vorliegen. Hierzu ist es erforderlich, die genannten Komponenten in einen geeigneten Träger einzuarbeiten.

Als Träger können z.B. auch pulverförmige Zubereitungen oder wäßrig-alkoholische Lösungen dienen, die als Mundwässer 0 bis 15 Gew..-% Ethanol, 1 bis 1,5 Gew.-% Aromaöle und 0,01 bis 0,5 Gew.-% Süßstoffe oder als Mundwasser-Konzentrate 15 bis 60 Gew.-% Ethanol, 0,05 bis 5 Gew.-% Aromaöle, 0,1 bis 3 Gew.-% Süßstoffe sowie ggf. weitere Hilfsstoffe enthalten können und vor Gebrauch mit Wasser verdünnt werden. Die Konzentration der Komponenten A, B und C muß dabei so hoch gewählt werden, daß nach Verdünnung die genannten Konzentrationsuntergrenzen bei der Anwendung nicht unterschritten werden.

Als Träger können aber auch Gele sowie mehr oder weniger fließfähige Pasten dienen, die aus flexiblen Kunststoffbehältern oder Tuben ausgedrückt und mit Hilfe einer Zahnbürste auf die Zähne aufgetragen werden. Solche Produkte enthalten höhere Mengen an Feuchthaltemitteln und Bindemitteln oder Konsistenzreglern und Polierkomponenten. Darüber hinaus sind auch in diesen Zubereitungen Aromaöle, Süßstoffe und Wasser enthalten.

Als Feuchthaltemittel können dabei z.B. Glycerin, Sorbit, Xylit, Propylenglycole, Polyethenylenglycole, insbesondere solche mit Molekulargewichten von 200 bis 800 verewendet werden.

Als Bindemittel und Konsistenzregler dienen z.B. natürliche und synthetische wasserlösliche Polymere wie Carragheen, Traganth, Guar, Stärke und deren nichtionogene Derivate wie z.B. Hydroxypropylguar, Hydroxyethylstärke, Celluloseether wie z.B. Hydroxyethylcellulose oder Methylhydroxypropylcellulose. Auch Agar-Agar, Xanthan-Gum, Pectine, wasserlösliche Carboxyvinylpolymere (z.B. Carbopol® -Typen), Polyvinylalkohol, Polyvinylpyrrolidon, höhermolekulare Polyethylenglycole (Molekulargewicht $10^3$ bis $10^6$D).

Weitere Stoffe, die sich zur Viskositätskontrolle eignen, sind Schichtsilikare wie z.B. Montmorillonit-Tone, kolloidale Verdickungskieselsäuren, z.B. Aerogel-Kieselsäure oder pyrogene Kieselsäuren.

Als Polierkomponenten können alle hierfür bekannten Poliermittel, bevorzugt aber Fällungs- und Gelkieselsäuren, Aluminiumhydroxid, Aluminiumoxid, Calciumpyrophosphat, Calciumhydrogenphosphat, Kreide, Hydroxylapatit, Natriumaluminiumsilikate, z.B. Zeolith A oder organische Polymere, z.B. Polymethacrylat eingesetzt werden.

Besonders bevorzugt sind dabei solche Poliermittel, die aufgrund ihrer Struktur und Zusammensetzung weniger adsorptiv für die kationischen, antimikrobiell wirksamen Stoffe sind. Als besonders geeignet haben sich z.B. solche Fällungskieselsäuren erwiesen, die durch eine gezielte Nachbehandlung mit gelösten Aluminium- oder Erdalkalisalzen eine verminderte spezifische Oberfläche aufweisen. Solche Fällungskieselsäuren sind z.B. aus DE 26 10 207 C3 bekannt. Auch Aluminiumhydroxid oder speziell behandeltes Dicalciumphosphat-dihydrat sind besonders gut geeignet.

Die erfindungsgemäße Zahnpaste kann durch Zugabe von Aromaölen und Süßungsmitteln in ihren organoleptischen Eigenschaften verbessert werden. Als Aromaöle kommen alle für Mund- und Zahnpflegemittel gebräuchlichen natürlichen und synthetischen Aromen in Frage. Natürliche Aromen können sowohl in Form der aus den Drogen isolierten etherischen Öle als auch der aus diesen isolierten Einzelkomponenten verwendet werden. Bevorzugt sollte wenigstens ein Aromaöl aus der Gruppe Pfefferminzöl, Krausenminzöl, Anisöl, Sternanisöl, Kümmelöl, Eukalyptusöl, Fenchelöl, Zimtöl, Nelkenöl, Geraniumöl, Salbeiöl, Pimentöl, Thymianöl, Majoranöl, Basilikumöl, Citrusöl, Gaultheriaöl oder eine oder mehrere daraus isolierte synthetisch erzeugten Komponenten dieser Öle enthalten sein. Die wichtigsten Komponenten der genannten Öle sind z.B. Menthol, Carvon, Anethol, Cineol, Eugenol, Zimtaldehyd, Caryophyllen, Geraniol, Citronellol, Linalool, Salven, Thymol, Terpinen, Terpinol, Methylchavicol und Methylsalicylat. Weitere geeignete Aromen sind z.B. Menthylacetat, Vanillin, Jonone, Linalylacetat, Rhodinol und Piperiton.

Außer den genannten Alkylglucosid-Tensiden können auch andere nichtionische, ampholytische und kationische Tenside enthalten sein. Insbesondere zur Solubilisierung der meist wasserunlöslichen Aromaöle kann ein nichtionogener Lösungsvermittler aus der Gruppe der oberflächenaktiven Verbindungen erforderlich sein. Besonders geeignet für diesen Zweck sind z.B. oxethylierte Fettsäureglyceride, oxethylierte Fettsäuresorbitanpartialester oder Fettsäurepartialester von Glycerin- oder Sorbitan-Oxethylaten. Lösungsvermittler aus der Gruppe der oxethylierten Fettsäureglyceride umfassen vor allem Anlagerungsprodukte von 20 bis 60 Mol Ethylenoxid an Mono- und Diglyceride von linearen Fettsäuren mit 12 bis 18 C-Atomen oder an Triglyceride von Hydroxyfettsäuren wie Oxystearinsäure oder Ricinolsäure. Weitere geeignete Lösungsvermittler sind oxethylierte Fettsäuresorbitanpartialester; das sind bevorzugt Anlagerungsprodukte von 20 bis 60 Mol Ethylenoxid an Sorbitanmonoester und Sorbitandiester von Fettsäuren mit 12 bis 18 C-Atomen. Ebenfalls geeignete Lösungsvermittler sind Fettsäurepartialester von Glycerin- oder Sorbitan-Oxethylaten; das sind bevorzugt Mono- und Diester von $C_{12}$-$C_{18}$-Fettsäuren und Anlagerungsprodukten von 20 bis 60 Mol Ethylenoxid an 1 Mol Glycerin oder an 1 Mol Sorbit.

Die erfindungsgemäßen Zahnpasten enthalten bevorzugt als Lösungsvermittler für gegebenenfalls enthaltene Aromaöle Anlagerungsprodukte von 20 bis 60 Mol Ethylenoxid an gehärtetes oder ungehärtetes Ricinusöl (d.h. an Oxystearinsäure- oder Ricinolsäure-triglycerid), an Glyverin-mono-- und/oder -distearat oder an Sorbitanmono- und/oder -distearat.

Weitere übliche Zusätze für die erfindungsgemäßen Mundpflegemittel sind z.B.

- Natriumfluorid, Zinnfluorid
- Pigmente, z.B. Titandioxid
- Farbstoffe
- pH-Stellmittel und Puffersubstanzen wie z.B. Natriumbicarbonat, Natriumcitrat, Natriumbenzoat
- wundheilende und entzündungshemmende Stoffe wie z.B. Allantoin, Harnstoff, Azulen bzw. Kamillenextrakt
- gegen Zahnstein wirksame Stoffe wie z.B. Organophosphonate, z.B. Hydroxyethandiphosphonate, Azacycloheptan-diphosphonat oder Natrium-pyrophosphat.

Weitere erfindungsgemäße Zubereitungen sind wäßrige Zubereitungen gemäß Anspruch 6.

Die folgenden Beispiele sollen den Erfindungsgegenstand näher erläutern:

**Prüfung der Anreicherung von Chlorhexidin-digluconat in der Zahn-Pellicle**

Zahn-Pellicle in einer Menge von 3 mg (Trockengewicht) wurde auf eine Petrischale mit einer Fläche von $10 cm^2$ gegeben und dort mit 0,5 ml der Prüflösung in Kontakt gebracht. Nach einer Einwirkungszeit von 1 Minute wurde durch ein colorimetrisches Verfahren (Extinktionsmessung) die Abnahme der Chlorhexidin-diglucomat-Menge aus der Prüflösung gemessen. Diese Abnahme entspricht der Adsorption des Chlorhexidins an der Pellicle. Aus der folgenden Tabelle ist die Zusammensetzung der wäßrigen Prüflösung (gelöste Stoffe in ppm) und die an der Pellicle adsorbierte Menge (CHX-Abnahme) in ppm zu entnehmen.

Es wurden folgende Abkürzungen benutzt:

APG =   Alkyl-$(C_{12}$-$C_{14})$-glucosid (1.3)
CHX =   1,1'-Hexamethylen-bis-(5-(4-chlorphenol)-biguanid)-digluconat (Chlorhexidin-digluconat)
MFP =   Dinatrium-monofluorophosphat $(Na_2PO_3F)$
NaF =   Natriumfluorid
CAB =   Cocoamidopropyl-betain

$$(RCONH\text{-}(CH_2)_3\text{-} \overset{(+)}{N}(CH_3)_2COO^{(\cdot)}$$

RCO = Kokosfettsäure-Acylrest.

Aus der Tabelle ist ersichtlich, daß nur die erfindungsgemäße Zubereitung gemäß Versuch 1, 2 und 3 zu einer Anreicherung des Chlorhexidin-digluconat an der Pellicle führt.

|  | V | V | V | 1 | 2 | 3 | V | V | V |
|---|---|---|---|---|---|---|---|---|---|
| CHX | 50 | 50 | 50 | 50 | 50 | 50 | 50 | 50 | 50 |
| APG | - | 83 |  | 83 | 41 | 83 | 83 | - |  |
| MFP | - |  | 310 | 310 | 310 | 151 | - | - | 310 |
| NaF | - |  |  |  |  |  | 91 | - |  |
| CAB | - |  |  |  |  |  |  | 166 | 166 |
| CHX- Abnahme | 4,8 | 7,9 | 7,9 | 25.2 | 10,6 | 12,3 | 7,4 | 6.2 | 2,3 |

**Anwendungsbeispiele**

| 1. Zahnpasten | 1.1 | 1.2 | 1.3 |
|---|---|---|---|
| $CaCO_3$ (Kreide, gefällt) | 40 | 35 | - |

(fortgesetzt)

| 1. Zahnpasten | 1.1 | 1.2 | 1.3 |
|---|---|---|---|
| Fällungskieselsäure. abrasiv | - | - | 15 |
| verdickende Kieselsäure | - | - | 5 |
| Sorbit | 5 | 3 | 5 |
| Glycerin | 5 | 10 | 10 |
| Chlorhexidin-digluconat | 0,05 | 0,1 | 0,05 |
| Aminfluorid 297 | 0,2 | 0,3 | - |
| Plantaren® 1200 | 0,1 | 0,3 | 0,2 |
| $Na_2PO_3F$ | 0,04 | 0,8 | 0,1 |
| Cremophor® RH60 | 0,2 | 0,3 | 0,2 |
| Aroma (Pfefferminzöl) | 0,1 | 0,15 | 0,1 |
| Saccharin-Natrium | 0,1 | 0,1 | 0,1 |
| Jaguar HP60 | 0,8 | 1,0 | 0,8 |
| Wasser | ad 100 | ad 100 | ad 100 |

| 2. Mundwässer (gebrauchsfertig) | 2.1 | 2.2 | 2.3 |
|---|---|---|---|
| Ethanol (99 %ig, unvergällt) | 5,0 | - | 10 |
| Chlorhexidin-digluconat | 0,03 | 0,05 | 0.015 |
| Plantaren 1200 | 0,05 | - | 0.03 |
| Plantaren 2000 | - | 0,08 | 0,02 |
| $Na_2PO_3F$ | 0,25 | 0,2 | 0,5 |
| Sorbit | 3,0 | - | 3,0 |
| Saccharin-Natrium | 0,05 | 0,05 | 0,05 |
| Cremophor® RH 60 | 0,1 | 0,1 | 0,1 |
| Aromaöl (Pfefferminzöl) | 0,1 | 0,1 | 0,2 |
| Farbstoff (blau, C.J. : 42090) | 0,1 | 0,1 | 0,1 |
| Wasser | ad 100 | ad 100 | ad 100 |

## 3. Mundwasserkonzentrate

| **Flüssige Konzentrate** | **3.1** 20-fach konzentriert | **3.2** 50-fach konzentriert |
|---|---|---|
| Ethanol | 40 | 40 |
| Plantaren 1200 | 2 | - |
| Plantaren 2000 | - | 20 |
| Chlorhexidin-digluconat | 0,5 | 1,0 |
| $Na_2PO_3F$ | 0,5 | 1,0 |
| Saccharin-Natrium | 1,0 | 2,5 |
| Cremophor RH60 | 2,0 | 5,0 |
| Aroma (Pfefferminzöl) | 2,0 | 5,0 |
| Farbstoff (blau, C.J. 42090) | 1,0 | 2,5 |
| Wasser | ad 100 | ad 100 |

| **Pulverförmiges, tablettierbares Mundwasserkonzentrat** | **3.3** Anwendung: 1 g pro 100 g Wasser |
|---|---|
| Sorbit (Pulver) | 50 |
| $NaHCO_3$ | 19 |
| Stärke (wasserlöslich) | 1 |
| Chlorhexidin-digluconat | 3 |
| Plantaren 1200 | 5 |
| $Na_2PO_3F$ | 2,5 |
| Cremophor RH 60 | 1,0 |
| Aromaöl | 1,0 |
| Citronensäure | 17,5 |

Es wurden folgende Handelsprodukte eingesetzt:

| Plantaren® 1200 | Alkylglucosid: RO$(C_6H_{10}O_5)_x$-H (50 Gew.-% in Wasser) R = $C_{12}$-$C_{16}$-Alkyl, x = 1,4 |
| Plantaren® 2000 | Alkylglucosid: RO$(C_6H_{10}O_5)_x$-H (ca. 50 Gew.-% in Wasser) R= $C_8$-$C_{16}$-Alkyl, x = 1,4 |
| Cremophor® RH60 | hydr. Rizinusölethylat (60 Mol EO) |
| Jaguar® HP60 | Hydroxypropyl-Guar |

Die Gew.-%-Angaben in den Tabellen beziehen sich auf wasserfreie Wirksubstanz.

**Patentansprüche**

1. Mund- und Zahnpflegemittel mit einem Gehalt an wasserlöslichen kationischen Wirkstoffen (A), **dadurch gekennzeichnet, daß** es zur Anreicherung dieser Wirkstoffe in den kariogenen Biofilmen der Mund- und Zahnoberfläche zusätzlich ein Alkyl-(oligo)-glucosid (B) der Formel RO$(C_6H_{10}O_5)_x$-H, in der R eine Alkylgruppe mit 8 bis 16 C-Atomen, $(C_6H_{10}O_5)$ ein Glucosidrest und x dessen Oligomerationsgrad von 1 bis 10 ist, sowie ein wasserlösliches Monofluorophosphat-Salz (C) enthält.

2. Mund- und Zahnpflegemittel nach Anspruch 1, **dadurch gekennzeichnet. daß** als wasserlösliches Monofluorophosphat-Salz das Natrium-monofluorophosphat enthalten ist.

3. Mund- und Zahnpflegemittel nach einem der Ansprüche 1 oder 2, **dadurch gekennzeichnet, daß** als kationischer antimikrobieller Wirkstoff eine Biguanidverbindung, bevorzugt Chlorhexidin, enthalten ist.

4. Mund- und Zahnpflebemittel nach einem der Ansprüche 1 bis 3, **dadurch gekennzeichnet, daß** als Alkyl-(oligo)-glycosid ein Alkyl-(oligo)-glucosid der Formel RO$(C_6H_{10}O_5)_x$-H, in der R eine Alkylgruppe mit 12 bis 14 C-Atomen ist und x einen Mittelwert von 1 bis 4 hat, enthalten ist.

5. Mund- und Zahnpflegemittel nach Anspruch 1 bis 4, **dadurch gekennzeichnet, daß** eine Kombination von

   (A) 0,004 bis 0,5 Gew.-% eines kationischen antimikrobiellen Wirkstoffs
   (B) 0,005 bis 1 Gew.-% eines Alkyl-(oligo)-glycosids und
   (C) 0,003 bis 1,2 Gew.-% eines Monofluorophosphat-Salzes (berechnet als Na-Salz)

   enthalten ist.

6. Wäßrige Zubereitungen, die wenigstens 40 ppm eines wasserlöslichen kationischen antimikrobiellen Wirkstoffs, wenigstens 50 ppm eines Alkyl-(oligo)-glucosids der Formel RO $(C_6H_{10}O_5)_x$-H, in der R eine Alkylgruppe mit 10 bis 16 C-Atomen, $(C_6H_{10}O_5)$ ein Glucosidrest und x dessen Oligomerisationsgrad von 1 bis 10 ist, und wenigstens 30 ppm eines Fluorphosphate-Ions (PO$_3$F$^{2(-)}$) in der Mundhöhle in gelöster Form zur Verfügung stellen, zur Behandlung kariogener Biofilme und Anreicherung wasserlöslicher kationischer Wirkstoffe in den Biofilmen.

**Claims**

1. Oral and dental care preparation containing water-soluble cationic active principles (A), **characterized in that**, to concentrate these active principles in the cariogenic biofilms of the mouth and tooth surface, it additionally contains an alkyl (oligo)glucoside (B) with the formula RO$(C_6H_{10}O_5)_x$-H, in which R is an alkyl group containing 8 to 16 carbon atoms, $(C_6H_{10}O_5)$ is a glucoside unit and x is its degree of oligomerization of 1 to 10, and a water-soluble monofluorophosphate salt (C).

2. Oral and dental care preparation as claimed in claim 1, **characterized in that** the water-soluble monofluorophosphate salt is sodium monofluorophosphate.

3. Oral and dental care preparation as claimed in claim 1 or 2, **characterized in that** the cationic antimicrobial agent is a biguanide compound, preferably chlorhexidine.

4.  Oral and dental care preparation as claimed in any of claims 1 to 3, **characterized in that** the alkyl (oligo)glycoside is an alkyl (oligo)glucoside with the formula $RO(C_6H_{10}O_5)_x$-H, in which R is an alkyl group containing 12 to 14 carbon atoms and x has a mean value of 1 to 4.

5.  Oral and dental care preparation as claimed in claims 1 to 4, **characterized in that** it contains a combination of

    (A) 0.004 to 0.5% by weight of a cationic antimicrobial agent,
    (B) 0.005 to 1% by weight of an alkyl (oligo)glycoside and
    (C) 0.003 to 1.2% by weight of a monofluorophosphate salt (expressed as Na salt).

6.  Water-based preparations which release at least 40 ppm of a water-soluble cationic antimicrobial agent, at least 50 ppm of an alkyl (oligo)glucoside with the formula $RO(C_6H_{10}O_5)_x$-H, in which R is an alkyl group containing 10 to 16 carbon atoms, $(C_6H_{10}O_5)$ is a glucoside unit and x is its degree of oligomerization of 1 to 10, and at least 30 ppm of a fluorophosphate ion $(PO_3F^{2(-)})$ in dissolved form in the oral cavity for treating cariogenic biofilms and concentrating water-soluble cationic active principles in the biofilms.

## Revendications

1.  Produit d'hygiène bucco-dentaire contenant des substances actives cationiques solubles dans l'eau (A), **caractérisé en ce qu'**il renferme en plus, pour concentrer ces substances actives dans les biofilms cariogènes de la surface de la bouche et des dents, un alkyl(oligo)glucoside (B) de la formule $RO(C_6H_{10}O_5)_x$-H, dans laquelle R représente un groupe alkyle comportant 8 à 16 atomes de C, $(C_6H_{10}O_5)$ correspond à un radical glucoside et x est son degré d'oligomérisation compris entre 1 et 10, ainsi qu'un sel de monofluorophosphate soluble dans l'eau (C).

2.  Produit d'hygiène bucco-dentaire selon la revendication 1, **caractérisé en qu'**il renferme comme sel de monofluorophosphate soluble dans l'eau, le monofluorophosphate de sodium.

3.  Produit d'hygiène bucco-dentaire selon une des revendications 1 ou 2, **caractérisé en qu'**il renferme comme substance active antimicrobienne cationique, un composé de biguanide, de préférence de la chlorhexidine.

4.  Produit d'hygiène bucco-dentaire selon une des revendications 1 à 3, **caractérisé en qu'**il renferme comme alkyl (oligo)glycoside, un alkyl-(oligo)-glucoside de la formule $RO(C_6H_{10}O_5)_x$-H, dans laquelle R représente un groupe alkyle comportant 12 à 14 atomes de C et x possède une valeur moyenne comprise entre 1 et 4.

5.  Produit d'hygiène bucco-dentaire selon une des revendications 1 à 4, **caractérisé en qu'**il renferme une association de

    (A) 0,004 à 0,5 % en poids d'une substance active antimicrobienne cationique
    (B) 0,005 à 1 % en poids d'un alkyl(oligo)glycoside et
    (C) 0,003 à 1,2 % en poids d'un sel de monofluorophosphate (calculé comme sel de sodium).

6.  Préparations aqueuses qui libèrent, sous forme dissoute dans la cavité buccale, au moins 40 ppm d'une substance active antimicrobienne cationique soluble dans l'eau, au moins 50 ppm d'un alkyl-(oligo)-glucoside de la formule $RO(C_6H_{10}O_5)_x$-H, dans laquelle R représente un groupe alkyle comportant 10 à 16 atomes de carbone, $(C_6H_{10}O_5)$ correspond à un radical glucoside et x est son degré d'oligomérisation compris entre 1 et 10, et au moins 30 ppm d'un ion de fluorophosphate $(PO_3F^{2(-)})$, pour traiter les biofilms cariogènes et concentrer les substances actives cationiques solubles dans l'eau dans les biofilms.